**Europäisches Patentamt**

**European Patent Office**

**Office européen des brevets**

(19)

(11) Numéro de publication : **0 248 719 B1**

# FASCICULE DE BREVET EUROPEEN

(12)

(45) Date de publication du fascicule du brevet :
**31.07.91 Bulletin 91/31**

(51) Int. Cl.$^5$ : **A61M 5/14**

(21) Numéro de dépôt : **87401210.7**

(22) Date de dépôt : **29.05.87**

(54) **Détecteur de bulles dans un circuit de liquide.**

(30) Priorité : **28.05.86 FR 8607657**

(43) Date de publication de la demande :
**09.12.87 Bulletin 87/50**

(45) Mention de la délivrance du brevet :
**31.07.91 Bulletin 91/31**

(84) Etats contractants désignés :
**AT BE CH DE ES GB GR IT LI LU NL SE**

(56) Documents cités :
**EP-A- 0 181 691**
**EP-A- 0 196 822**
**DE-A- 2 716 714**
**US-A- 4 100 797**
**US-A- 4 366 384**
**US-A- 4 487 601**

(73) Titulaire : **Société M.M.S.**
**Zone Indusnor Lotissement de l'Echangeur**
**F-64000 Pau (FR)**

(72) Inventeur : **Siretchi, Roman Résidence Les**
**Jardins de Trespoey**
**4, rue des Hauts-Champs de Trespoey**
**F-64000 Pau (FR)**
Inventeur : **Vignacq, Pierre**
**5, avenue du Lac**
**F-40140 Soustons (FR)**

(74) Mandataire : **Joly, Jean-Jacques et al**
**CABINET BEAU DE LOMENIE 55, rue**
**d'Amsterdam**
**F-75008 Paris (FR)**

**Description**

La présente invention concerne un détecteur de bulles ou, d'une façon générale, un détecteur de discontinuité de liquide dans un circuit de liquide. Plus particulièrement, l'invention est relative à un détecteur de bulles du type comportant des moyens photoémetteurs et des moyens photorécepteurs destinés à être placés de deux côtés opposés d'une conduite parcourue par le liquide, et des moyens de traitement de signaux fournis par les moyens photorécepteurs afin de produire un signal indicateur en réponse à l'apparition d'une bulle ou discontinuité de liquide dans la conduite au niveau du détecteur.

L'invention est notamment, mais non exclusivement, utilisable pour la détection de bulles d'air dans des circuits de liquide de systèmes de nutrition parentérale ou de transfusion sanguine, ainsi que pour la détection de fin de flacon dans de tels systèmes.

Dans les systèmes de nutrition parentérale ou de transfusion sanguine, le liquide provenant d'un flacon est injecté à un patient au moyen d'une pompe. De l'air peut être introduit dans le circuit de liquide, par exemple si la pompe continue à fonctionner après vidage du flacon, ou en cas de perte d'étanchéité de la tubulure de transfusion ou perfusion parentérale par suite des efforts exercés par la pompe. En raison du danger qu'elles représentent pour le patient, les bulles doivent être détectées.

L'utilisation d'un détecteur de bulles inclus dans la tubulure de perfusion ou transfusion n'est pas envisageable pratiquement et économiquement. En effet, la tubulure doit être stérélisable et doit pouvoir facilement être démontée et remontée ; de plus, le détecteur ne doit pas transmettre du courant électrique au corps du patient via le liquide qui peut être très conducteur ; enfin, en tant que produit consommable, la tubulure doit être d'un prix aussi réduit que possible.

Il est donc nécessaire d'utiliser des détecteurs de bulles extérieurs à la ligne de perfusion ou transfusion, ce qui rend la détection assez délicate, d'autant que les liquides peuvent être très divers et présenter des caractéristiques très différentes.

Parmi les détecteurs de bulles connus, certains sont de type optique avec un photoémetteur et un photorécepteur placés face à face de deux côtés opposés d'une conduite en matériau transparent parcourue par le liquide. Le passage d'une bulle provoque une variation de la transparence du milieu situé entre le photoémetteur et le photorécepteur, d'où une variation qui peut être détectée d'un signal fourni par le photodétecteur.

L'utilisation de ces détecteurs connus soulève une difficulté en raison de la très grande dispersion de la transparence des liquides pouvant parcourir la conduite : liquides transparents, translucides ou presque opaques.

Pour résoudre cette difficulté, il a été proposé des détecteurs de bulles de type optique avec préréglage manuel de la valeur de référence par rapport à laquelle est analysée la variation du signal fourni par le photodétecteur. Mais le détecteur ainsi réglé n'est capable de fonctionner correctement que dans une plage limitée de la transparence du liquide. De plus, une erreur de réglage peut toujours survenir avec des conséquences graves.

Il a encore été proposé dans la demande de brevet français No 2361644 d'utiliser plusieurs couples émetteurs-récepteurs optiques dont l'un est utilisé pour effectuer un réglage automatique ou préconditionnement du détecteur en distinguant entre liquide transparent et liquide opaque. Il reste que, pour chaque régime de fonctionnement (liquide "transparent" ou liquide "opaque"), la plage de transparence reste étendue ce qui rend toujours délicat le choix d'une valeur de référence. De plus, ce réglage automatique est réalisé au prix d'une assez grande complication du détecteur.

Enfin, le brevet US-A-4366384 décrit un détecteur de bulles avec deux ensembles émetteurs-récepteurs optiques espacés l'un de l'autre le long de la conduite. Dans chaque ensemble, il y a deux récepteurs, l'un, opposé à l'émetteur, détectant la lumière ayant traversé le liquide et l'autre, décalé par rapport au premier, détectant la lumière réfléchie par l'intérieur de la conduite. Une bulle d'air est signalée lorsque les sorties des deux détecteurs indiquent la détection d'une quantité de lumière importante, ce qui rend le fonctionnement de l'appareil indépendant de la transparence ou de l'opacité du liquide. L'utilisation de deux ensembles permet, en ne retenant que la détection d'une bulle d'air simultanément par ceux-ci, d'éviter des détections parasites pour des petites bulles.

La présente invention a pour but de fournir un détecteur de bulles ou de discontinuité de liquide capable de fonctionner de façon sûre dans une large plage de transparence de liquide, sans nécessiter de réglage fonction du liquide et tout en conservant une structure relativement simple.

Ce but est atteint au moyen d'un détecteur du type défini en tête de la présente description et dans lequel, conformément à l'invention :

— les moyens photoémetteurs et photorécepteurs comprennent un premier et un deuxième couple émetteurs-récepteurs optiques destinés à être espacés l'un de l'autre le long de la conduite et un circuit de régulation associé à chaque couple émetteur-récepteur pour faire fonctionner celui-ci dans une plage de sensibilité optimale indépendamment de la transparence du liquide, le circuit de régulation étant agencé pour alimenter l'émetteur sous une tension qui varie comme la différence entre une tension fournie par le récepteur et une tension de référence, et

— les moyens de traitement comprennent un circuit différentiel agencé de manière à fournir un signal (Uo) représentatif de la différence entre les signaux fournis par les récepteurs et un circuit comparateur relié au circuit différentiel pour fournir ou non ledit signal indicateur en fonction de l'amplitude du signal différentiel (Uo).

La combinaison de la détection différentielle avec le réglage automatique de la sensibilité à sa valeur optimale permet ici de travailler dans une large plage de transparence de liquide, dans les meilleures conditions possibles et, sans demander de pré-réglage particulier fonction de cette transparence. Il en résulte une plus grande sécurité de fonctionnement du détecteur de bulles, ce qui est particulièrement important dans le cadre de l'application aux systèmes de perfusion ou transfusion.

De plus, le détecteur conforme à l'invention est utilisable aussi bien pour détecter des bulles dans une conduite parcourue par un liquide que pour détecter le vidage complet d'un réservoir ou flacon alimentant un circuit de liquide, le vidage de la conduite sur laquelle le détecteur est monté étant, pour le détecteur, équivalent à l'apparition d'une bulle d'air.

Aussi, le détecteur conforme à l'invention est-il utilisable en particulier comme détecteur de fin de flacon dans des systèmes de perfusion ou transfusion. Dans le cadre de cette application, le détecteur conforme à l'invention présente divers avantages par rapport aux détecteurs de fin de flacon connus. Ces derniers sont généralement des dispositifs de type optique agencés pour réaliser une surveillance de l'écoulement du liquide dans la chambre à gouttes ; le détecteur est activé au passage de chaque goutte et une alarme de fin de flacon est donnée lorsque l'intervalle de temps entre deux gouttes dépasse un seuil prédéterminé. Mais le système de détection est alors sensible au débit d'alimentation en liquide puisque la fréquence des gouttes varie selon le débit. Par ailleurs, le positionnement du détecteur par rapport à la chambre à gouttes est imprécis et facilement déréglable. De plus, l'émetteur et le récepteur sont relativement éloignés l'un de l'autre, puisque la chambre à gouttes a un diamètre de plusieurs centimètres, d'où de possibles anomalies de fonctionnement par suite de changement de l'éclairage de l'environnement (soleil, lumière artificielle,...). L'utilisation d'un détecteur conforme à l'invention au niveau de la conduite alimentant la chambre à gouttes permet d'éviter tous ces inconvénients : la détection est insensible au débit et à la nature du fluide et n'est pas affectée par des changements de l'éclairage environnant puisque la distance entre émetteur et récepteur associés est de quelques mm seulement.

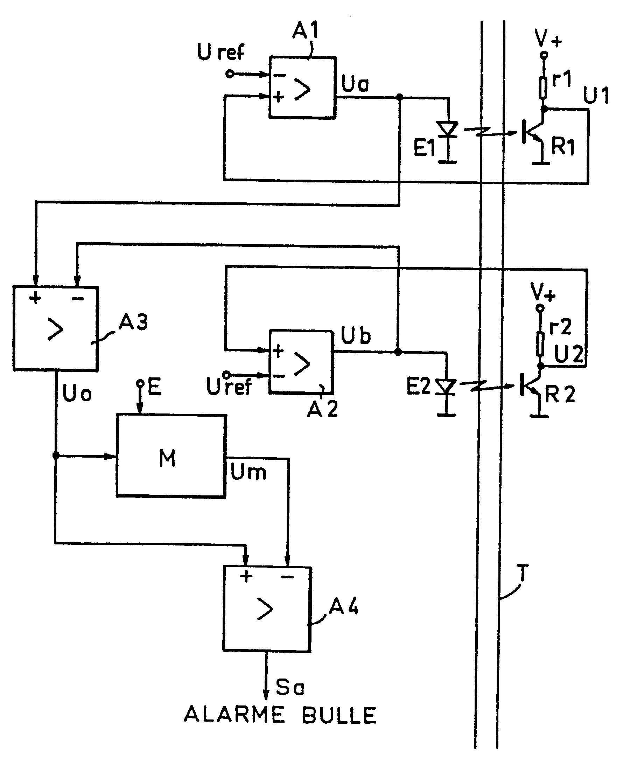

D'autres particularités et avantages du détecteur conforme à l'invention ressortiront à la lecture de la description faite ci-après, à titre indicatif mais non limitatif, en référence au dessin annexé dont la figure unique est un schéma d'un mode de réalisation d'un tel détecteur.

Le détecteur illustré sur cette figure est associé à une conduite ou tubulure, par exemple une tubulure faisant partie d'un système de nutrition parentérale ou de transfusion sanguine dans lequel le liquide est administré à un patient en utilisant une pompe, notamment une pompe de type péristaltique. Le détecteur est alors placé sur une tubulure située en aval de la pompe.

Le détecteur comprend deux couples émetteurs-récepteurs optiques respectivement E1-R1 et E2-R2. Dans chaque couple, l'émetteur E1, E2 et le récepteur correspondant R1, R2 sont situés face à face, de part et d'autre de la tubulure T, à l'extérieur et à proximité de la paroi transparente de celle-ci. Les deux couples E1-R1, E2-R2 sont espacés l'un de l'autre d'une distance par exemple égale à quelques millimètres.

Les couples E1-R1 et E2-R2 sont de préférence identiques. Les émetteurs E1, E2 sont par exemple des diodes électroluminescentes émettant dans l'infrarouge tandis que les récepteurs R1, R2 sont des phototransistors sensibles au rayonnement émis par les diodes. Chaque transistor R1, R2 a son circuit émetteur-collecteur branché en série avec une résistance r1, r2 entre la masse et une borne portée au potentiel V+ d'une source d'alimentation. Les tensions U1, U2 prélevées entre la masse et les collecteurs des transistors R1, R2 constituent les signaux de sortie de ceux-ci, signaux dont l'amplitude varie en fonction de l'intensité de l'éclairement reçu.

Les diodes E1, E2 sont alimentées sous des tensions Ua, Ub délivrées par des amplificateurs différentiels A1, A2. Ces derniers ont leurs entrées négatives reliées à une borne portée à un potentiel de référence Uref et leurs entrées positives reliées aux collecteurs des transistors R1, R2 pour recevoir les tensions U1, U2, respectivement.

Les tensions Ua et Ub sont par ailleurs appliquées aux entrées positive et négative d'un amplificateur différentiel A3 qui délivre un signal différentiel Uo. Celui-ci est appliqué à l'entrée d'une mémoire analogique M, par exemple un circuit à condensateur, pour mémoriser l'amplitude du signal différentiel Uo en réponse à l'application d'un signal de commande sur une entrée E de commande de mémorisation dans la mémoire M. En parallèle, le signal différentiel Uo est appliqué de façon continue sur une entrée d'un comparateur A4 dont l'autre entrée reçoit la valeur mémorisée Um du signal Uo en sortie de la mémoire M. Le comparateur A4 délivre un signal d'alarme Sa lorsque la différence entre les signaux Uo et Um dépasse un seuil prédéterminé.

En variante, on pourra utiliser une mémoire M de type numérique. Le signal Uo est alors converti sous forme numérique en sortie de l'amplificateur A3 et le comparateur A4 est réalisé sous forme d'un circuit numérique.

Le fonctionnement du détecteur qui vient d'être décrit est le suivant.

Dans chaque couple émetteur-récepteur, lorsque la transparence du liquide varie, une variation correspondante de la tension fournie par le phototransistor se produit, variation qui se traduit par une modification de la tension appliquée à la diode électroluminescente, c'est-à-dire une modification de la puissance d'émission. Par exemple, si la transparence diminue, l'amplitude du signal fourni par le phototransistor diminue, mais la tension d'alimentation de la diode augmente par suite de l'augmentation de la différence entre la tension de référence Uref et celle fournie par le phototransistor. Il est ainsi possible de maintenir le fonctionnement du phototransistor dans une plage linéaire de sensibilité maximale pour conserver une sensibilité maximale de détection. En d'autres termes, la réinjection du signal fourni par chaque détecteur sur une entrée de l'amplificateur différentiel qui fournit la tension d'alimentation de l'émetteur correspondant permet de réguler la sensibilité du couple émetteur-récepteur pour maintenir cette sensibilité à la valeur optimale.

L'amplificateur différentiel A3 fournit un signal différentiel Uo proportionnel à la différence entre les signaux Ua et Ub, donc représentatif de la différence entre les tensions U1 et U2.

En l'absence de bulles d'air dans la tubulure T, les transparences des milieux situés dans les domaines d'action des couples émetteurs-récepteurs sont identiques. Le signal Uo a une amplitude faible sinon nulle. Si, d'une part, les couples émetteurs-récepteurs et d'autre part, les amplificateurs A1 et A2 ont des caractéristiques identiques ou très voisines L'apparition d'une bulle d'air au niveau d'un couple émetteur-récepteur va donc entraîner une augmentation importante de l'amplitude du signal Uo en valeur absolue.

Mais il n'est pas indispensable d'avoir des émetteurs récepteurs ou des amplificateurs A1, A2 ayant des caractéristiques identiques puisque la détection de bulles d'air est réalisée non pas en comparant la valeur absolue instantanée du signal Uo à une valeur de seuil, mais en surveillant les variations d'amplitude du signal Uo.

Ainsi, un intervalle de temps prédéterminé après le début du fonctionnement du détecteur, par exemple une dizaine de secondes, la valeur du signal Uo est stockée dans la mémoire M par envoi d'un signal de commande de mémorisation sur l'entrée E. Pendant le reste du fonctionnement, la valeur Um mémorisée est utilisée comme valeur de référence pour détecter une variation du signal instantané Uo telle que la différence Uo-Um dépasse un seuil prédéterminé. Une telle variation sera alors imputée à l'apparition d'une bulle d'air et provoquera l'émission d'un signal d'alarme.

Comme déjà indiqué, le détecteur est aussi utilisable pour la détection de fin de flacon dans des systèmes de perfusion ou transfusion. Dans ce cas, le vidage de la tubulure au niveau du détecteur est assimilé au passage d'une bulle d'air. Le détecteur est alors placé obligatoirement sur une tubulure en aval du flacon et en amont de la chambre à gouttes.

## Revendications

1. Détecteur de bulles ou de discontinuité de liquide dans un circuit de liquide, comportant des moyens photoémetteurs et des moyens photodétecteurs destinés à être placés de deux côtés opposés d'une conduite parcourue par le liquide, et comprenant un premier et un deuxième couple émetteurs-récepteurs optiques (E1-R1, E2-R2) destinés à être espacés l'un de l'autre le long de la conduite (T), et des moyens de traitement de signaux fournis par les moyens photorécepteurs pour produire un signal indicateur en réponse à l'apparition d'une bulle ou discontinuité de liquide dans la conduite au niveau du détecteur, caractérisé en ce que :

    — les moyens photoémetteurs et photorécepteurs comprennent en outre un circuit de régulation (A1, A2) associé à chaque couple émetteur-récepteur pour faire fonctionner celui-ci dans une plage de sensibilité optimale indépendamment de la transparence du liquide, le circuit de régulation (A1, A2) étant agencé pour alimenter l'émetteur (E1, E2) sous une tension (Ua, Ub) qui varie comme la différence entre une tension (U1, U2) fournie par le récepteur et une tension de référence (Uref), et

    — les moyens de traitement comprennent un circuit différentiel (A3) agencé de manière à fournir un signal (Uo) représentatif de la différence entre les signaux (U1, U2) fournis par les récepteurs (R1, R2) et un circuit comparateur (A4) relié au circuit différentiel (A3) pour fournir ou non ledit signal indicateur en fonction de l'amplitude du signal différentiel (Uo).

2. Détecteur selon la revendication 1, caractérisé en ce que le signal différentiel est proportionnel à la différence entre les tensions (Ua, Ub) d'alimentation des émetteurs.

3. Détecteur selon l'une quelconque des revendications 1 et 2, caractérisé en ce qu'il comprend une mémoire (M) pour mémoriser le signal différentiel à un instant donné, et un comparateur pour comparer la valeur instantanée (Uo) du signal différentiel à la valeur précédemment mémorisée (Um) du signal différentiel pour fournir le signal indicateur lorsque la différence entre lesdites valeurs instantanée (Uo) et mémorisée (Um) dépasse un seuil prédéterminé.

4. Dispositif pour la détection de fin de flacon dans une installation de perfusion ou transfusion, caractérisé en ce qu'il est constitué par un détecteur selon l'une quelconque des revendications 1 à 3.

**Patentansprüche**

1. Detektor für Blasen oder Diskontinuitäten einer Flüssigkeit in einem Flüssigkeitskreislauf mit
— zur Anordnung auf zwei entgegengesetzten Seiten einer von der Flüssigkeit durchströmten Leitung bestimmten Photoemitter- und Photodetektoreinrichtungen,
— einem ersten und einem zweiten, zur Anordnung entlang der Leitung (T) mit gegenseitigem Abstand bestimmten Opto-Sender/Empfänger-Paar (E1-R1, E2-R2)
und
— einer Verarbeitungseinrichtung für von den Photoempfängern gelieferte Signale zum Erzeugen eines Anzeigesignals für das Auftreten einer Blase oder Diskontinuität in der Leitung auf der Höhe des Detektors, dadurch gekennzeichnet, daß die Photoemitter- und Photodetektoreinrichtungen außerdem eine Regelschaltung (A1, A2) aufweist, die mit jedem Sender/Empfänger-Paar gekoppelt ist, um dieses unabhängig von der Transparenz der Flüssigkeit in einem optimalen Empfindlichkeitsbereich arbeiten zu lassen, wobei die Regelschaltung (A1, A2) betrieben wird, um den Sender (E1, E2) mit einer Spannung (Ua, Ub) zu speisen, die wie die Differenz zwischen einer von dem Empfänger gelieferten Spannung (U1, U2) und einer Bezugsspannung (Uref) variiert, und daß die Verarbeitungseinrichtung eine Differenzschaltung (A3), die so betrieben wird, daß sie ein Signal (Uo) liefert, das die Differenz zwischen den von den Empfängern (R1, R2) gelieferten Signalen wiedergibt, und eine Komparatorschaltung (A4) aufweist, die mit der Differenzschaltung (A3) verbunden ist, um in Abhängigkeit von der Amplitude des Differenzsignals (Uo) das Anzeigesignal abzugeben oder nicht.

2. Detektor nach Anspruch 1, dadurch gekennzeichnet, daß das Differenzsignal proportional ist zu der Differenz zwischen den Speisespannungen (Ua, Ub) für die Sender.

3. Detektor nach irgendeinem der Ansprüche 1 und 2, dadurch gekennzeichnet, daß er einen Speicher (M) zum Speichern des Differenzsignals in einem vorgegebenen Zeitpunkt und einen Komparator zum Vergleichen des Momentanwertes (Uo) des Differenzsignals mit dem zuvor gespeicherten Wert (Um) des Differenzsignals aufweist, um das Anzeigesignal abzugeben, wenn die Differenz zwischen dem Momentanwert (Uo) und dem gespeicherten Wert (Um) eine vorgegebene Schwelle überschreitet.

4. Vorrichtung zum Erkennen der Flakonentleerung in einer Perfusions- oder Transfusionseinrichtung, dadurch gekennzeichnet, daß sie durch einen Detektor nach einem beliebigen der Ansprüche 1 bis 3 gebildet ist.

**Claims**

1. Detector of air bubbles or of liquid discontinuity in a circuit of liquid, comprising photoemissive means and photoreceiver means adapted to be placed on two opposite sides of a conduit carrying the liquid, and comprising first and second optical emitter-receiver means (E1-R1, E2-R2) designed to be placed apart one from the other along the conduit (T), and means for processing the signals produced by the photoreceiver means in order to produce an informative signal in response to the appearance of an air bubble or a discontinuity of liquid in the conduit at the detector level, characterized in that :
— the photo-emissive and photo-receiver means further comprise a regulator circuit (A1, A2) operationally coupled to each emitter-receiver pair for controlling the operation of said pair inside a maximum response range, independently of the transparency of the liquid, the regulator circuit (A1, A2) being so arranged as to supply the emitter (E1, E2) with a voltage (Ua, Ub) which is a function of the difference between a voltage (U1, U2) supplied by the receiver and a reference voltage (Uref), and
— the processing means comprise a differential circuit (A3), arranged so as to produce a signal (Uo) representing the difference between the signals (U1, U2) produced by the receivers (R1, R2) and a comparator circuit (A4) connected to the differential circuit (A3) in order to supply or not, said informative signal as a function of the amplitude of the differential signal (Uo).

2. Detector according to claim 1, characterized in that the differential signal is proportional to the difference between the emitters supply voltages (Ua, Ub).

3. Detector according to any one of claims 1 and 2, characterized in that it comprises a memory (M) for storing the differential signal at a given time, and a comparator for comparing the instant value (Uo) of the differential signal to the precedingly stored value (Um) of the differential signal in order to produce the informative signal when the difference between said instant (Uo) and stored (Um) values exceeds a predetermined threshold.

4. Device for detecting an empty bottle in a perfusion or transfusion installation, characterized in that it is constituted by a detector according to any one of claims 1 to 3.

ALARME BULLE